Europäisches Patentamt

European Patent Office

Office européen des brevets

(19)

(11) Publication number : **0 190 801**
**B1**

(12)

# EUROPEAN PATENT SPECIFICATION

(45) Date of publication of patent specification :
01.02.89

(51) Int. Cl.⁴ : **C 12 M   1/00**

(21) Application number : 86200148.4

(22) Date of filing : 03.02.86

(54) A process for carrying out microbiological fermentations in a device comprising a fixed system of an open cell foam as well as a device for carrying out such processes.

(30) Priority : 06.02.85 NL 8500332

(43) Date of publication of application :
13.08.86 Bulletin 86/33

(45) Publication of the grant of the patent :
01.02.89 Bulletin 89/05

(84) Designated contracting states :
AT CH DE FR GB IT LI LU NL SE

(56) References cited :
EP--A-- 0 046 901
EP--A-- 0 104 525
EP--A-- 0 130 116
FR--A-- 2 051 381
FR--E--    94 413
GB--A-- 1 281 874
GB--A-- 2 035 284
US--A-- 3 618 778

(73) Proprietor : RECTICEL
12, Avenue de Broqueville, Sint Pieters Woluwé
B-1150 Brussels (BE)

(72) Inventor : Verstraete, Willy
Dasstraat 4
B-9030 Wondelgem (BE)
Inventor : Deryke, Dirk
Hundelgemsesteenweg 586/33
B-9220 Merelbeke (BE)
Inventor : Creyf, Hubert
Leykendreef 5
B-8201 St.-Michiels-Brugge (BE)

(74) Representative : van der Beek, George Frans et al
Nederlandsch Octrooibureau Johan de Wittlaan 15
P.O. Box 29720
NL-2502 LS The Hague (NL)

## Description

The invention relates to a process for carrying out microbiological fermentations, wherein a nutrient liquid and, if necessary, gases are passed through a fixed system of open cell polyurethane foam units as well as a device for carrying out microbiological fermentations.

In European Patent Application 0046901 such a process for the anaerobic biological purification of waste water (sewage) containing organic contaminations is disclosed. Said purification is carried out by means of anaerobic microorganisms adhered to a polyurethane foam carrier, through which at least a part of the sewage is passed.

Due to the use of a macroporous material, having the shape of a large block or having been shaped into pieces having diameters of 1 to 5 cm, containing open macro pores and having a low density, as a carrier, a large active surface is offered to the microorganisms that grow only very slow, on which surface said microorganisms may be deposited uniformly and in a fixed state. Consequently, the bacteria cannot be flushed out to such a degree, that the capacity of the reactor is reduced or even stopped. Here the bacteria are forced to a decentralised growth, due to the macropores of the carrier, due to which a much larger substance exchange surface than in other processes is attained.

It appears from the publication of P. Huysman et al. in « Biotechnology Letters », Vol. 5, No. 9, 643-648 (1983), that in addition to the surface roughness and the dimensions of the pores, also the reticulation of foams, especially polyurethane foams which are used as packing materials in biological methane reactors, are of importance for colonisation of the active microorganisms.

Further, according to said authors, the biological transformation proceeds slower as the packing material is present in larger units. Especially in case a large block packing material is used the biological transformation proceeds slowly.

Although the known processes for microbiological fermentations provide reasonable yields, especially if in such processes fixed or non-fixed foam carriers are used, the results provided by said known processes and devices may be improved considerably.

In this connection it is still observed, that in using small foam cubes, these will start to float and, consequently, it is difficult to keep them in the reactor unless additional mechanical aids, such as a wire net, are mounted in the reactor. In addition, it appears that the contact of such cubes with liquid is hard to maintain.

Of course also the position of said cubes cannot be indicated as regards to the quantity because one does not know the position of such cubes at a defined time.

It seems useful to mention here that European patent application 104525 discloses a device for the biological purification of waste water, wherein waste water is fed to a collection of unembossed blocks of an open cell foam material.

Further European patent application 130116 discloses a transparant wall from a polyurethane foam, optionally containing microorganisms. Said wall contains pores, wherein microorganisms have been distributed, and said pores are closed at one side, so that said wall is impermeable for water and gas while the opposit side of said wall shows an open porosity. Such walls can be used in devices for microbiological processes wherein metabolites produced by microorganisms supplied may be isolated.

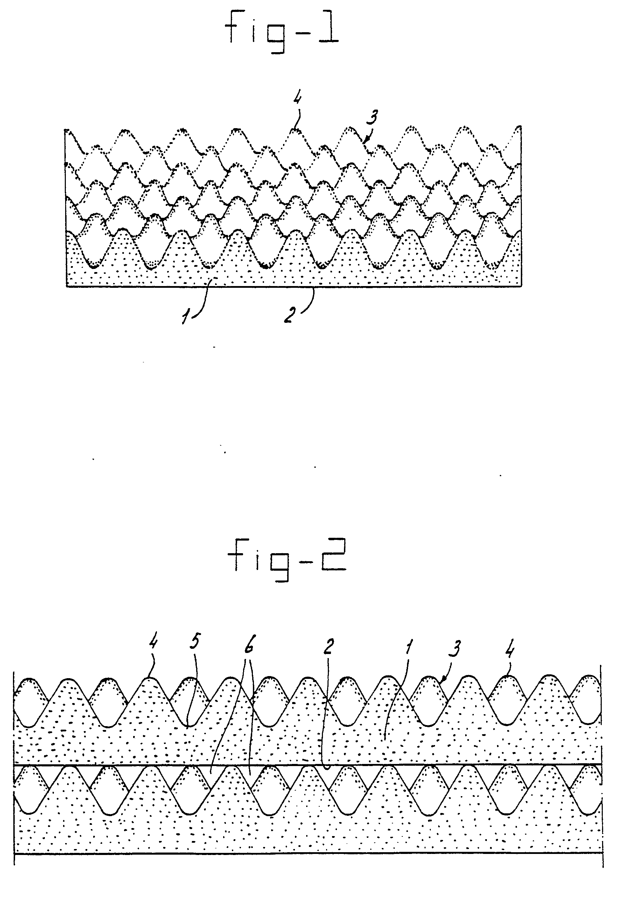

It was found now, that the yields may be improved by 20 to 25 % if in carrying out microbiological fermentations wherein a nutrient liquid and, if required, gases are passed through a fixed system of reticulated open cell polyurethane foam units, a system is used, that consists of at least two subsequent foam layers each having a flat main surface and an embossed main surface the latter consisting of a pattern of protractions and recessed parts of substantially equal dimensions as set forth in Figure 1, wherein in the subsequent layers the main surfaces are in a substantially parallel, respectively concentric position in respect of each other.

The used carrier of polyurethane foam may consist of a hard, a soft as well as a semi-hard foam. However, a soft or semi-hard foam having a dimensional stability, is preferred, so that even during the operation of the process with said material, the pores through which gas and liquid may flow, are maintained. With respect to the pore sizes of the foam it is observed, that generally this will amount 15 to 100 pores per inch (ppi) (2 to 200 pores per cm). The foam layers may be fixed in each known chemical and/or mechanical way.

In the process according to the invention a system of foam layers at least comprising two vertically mounted foam panels, which system is distributed over 10 to 100 %, in particular 40 to 80 % by volume, of the reactor capacity is preferred.

However, in some fermentations it is very efficient to use a system consisting from a rolled-up foam band (strip), in which the surface is preferably directed outwardly.

In the process according to the invention the concentration of the biomass is kept at an optimum level whereas the time during which the biomass is used, and, consequently, the yield of the reactions as well as the type of the reactions may be determined and controlled.

The present invention provides a process wherein a biological material is cultivated in an added nutrient, in which the nutrients and the other liquid and/or gaseous substances, required for the reaction are passed through a bed comprising a support layer for the biological material that is present in a reactor. The system is constructed in such a way that in addition to the movement of substances, microbes and enzymes through the open pores (micro openings) of the polyurethane foam also macro openings are present, through which the gases evolved during the reaction may escape easily.

The process according to the invention is particularly proper to be used in an upflow reactor, in which a system of adjacent foam layers is used wherein the flat surfaces are positioned in the flow direction.

According to the process of the invention aerobic, anaerobic as well as optionally aerobic microbiologic reactions may be carried out. Said reactions may be performed in the presence of bacteria, yeasts and/or fungi. They may be carried out also in absence of said microorganisms but in the presence of enzymes required for such a reaction.

Just like in the known processes wherein foam carriers are used for the microbes and/or enzymes, the biological material grows on the basis of an added nutrient.

The foam units may be used in microbiological processes of two types, viz.

(a) processes wherein the desired endproduct is dissolved in the liquid, which is evolved from the basis of a fermentation operation and wherein desirably the flushing out of active growing cells in the nutrient liquid is desired ;

(b) reactions wherein the desired finalproduct is present in the growing cell material and wherein the growing bactera or fungi cells are removed from the foam and are washed in a collector at the end of the fermentation in order to separate them from the liquid.

Examples of microbiological reactions of type (a) are the preparation of acetic acid from alcoholic solutions, of lactic acid, propionic acid, butyric acid, succinic acid, citric acid, acetyl methy carbinol, butylene glycol, gluconic acid, fumaric acid and oxalic acid from sugars.

Examples of microbiological reactions of type (b) are the preparation of yeasts, on molasses and liquified parts of wood pulp.

A very important use of the process according to the invention is also the treatment of waste water with aerobic or anaerobic sludge. The cell structure of the polyurethane foam system according to the invention prevents that bacteria involved in the anaerobic fermentation will form a compact layer which may cause obstructions in the reactor.

The process according to the invention has the advantage that a controllable system is provided, that may be applied easily and wherein the amount of liquid may be adjusted. In order to prevent the formation of preferred channels in the intermediate spaces between the rolled packages various layers of rolled packages are placed on each other in a staggered position. By using such rolled-up foam bands the diffusion distance may be controlled and, consequently, optimalized.

In addition, the spaces between the protruding naps may be used for gas evolution.

The distance from any point in the foam bands to the aqueous phase being exposed to the free mixing and flowing is preferably always smaller than 10 cm.

The invention also relates to a device for carrying out microbiological fermentations. Here in a vessel, being provided with means to supply nutrient liquid and gases, and means to discharge liquid, gas and, optionally, cells or microbes, a system of units of polyurethane foam layers having open pores is fixed, wherein the polyurethane foam layers each have a flat main surface and an embossed main surface the latter consisting of a pattern of protractions and recessed parts of substantially equal dimensions as set forth in Figure 1, wherein the main surfaces are positioned in a substantially parallel and concentric position respectively in respect of each other.

The carrier system of polyurethane foam units preferably consists of at least two adjacent foam panels.

Very suitably the carrier system of polyurethane foam units may also consist of a rolled-up foam band, wherein the embossed surface is directed outwardly whereas the axis of the rolled-up band is positioned substantially in the flow direction.

A vessel wherein proper foam units may be applied is an elongated cylindrical vessel having a substantially vertical axis, wherein polyurethane foam units have been staggered uniformly whereas the supply lines for a fresh nutrient liquid and gases may be present at any desired level.

The invention is further elucidated by means of the further description referring to the three figures.

Fig. 1 is a perspective view of an open cell polyurethane foam (1) the main surface (2) of which is flat and the main surface (3) of which is embossed. The embossed surface has a regular pattern of protruding parts (4) and recessed parts (5) having substantially equal dimensions.

Fig. 2 is a side view of a two-layered system of an open cell polyurethane foam wherein (4) indicates a protruding part and (5) a recessed part whereas (6) indicates openings between the flat surface (2) and the embossed surface (4).

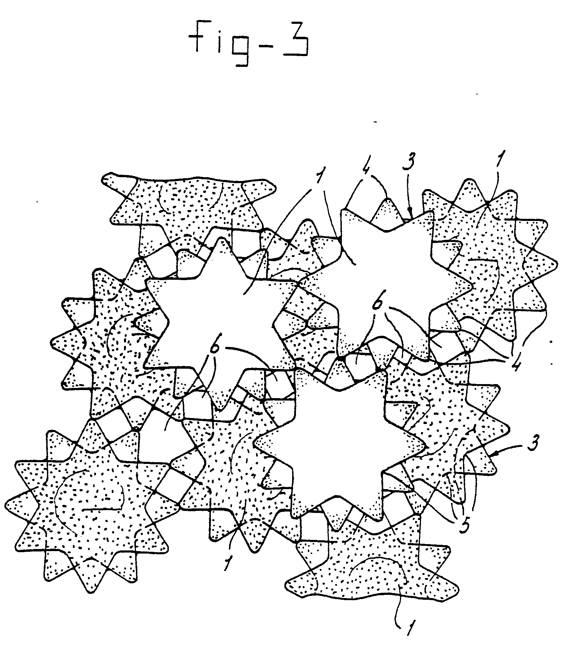

Fig. 3 is a schematic top view of adjacently applied rolls of foam band having one flat main surface and one embossed surface, wherein the embossed surfaces are directed outwardly. On one layer of the adjacently applied rolls of foam bands at least one other layer of adjacently applied rolls of foam bands are positioned, which last rolls are each staggered in respect of the underlaying rolls. In this figure the openings (7) are openings between subsequent layers.

The following examples further illustrate some preferred embodiments of the present invention. These examples are illustrations of some features of the present invention.

Example I

Waste water of a starch processing plant was tested on its capacity to be decomposed anaerobically in case the following two systems are used.

a) an anaerobic reactor (as described in « Proceedings of AWWT », Noordwijkerhout, November 23-25, 1983, P. Huysman, P. van Meenen, P. van Assche and W. Verstraete : fig. 1) was filled with reticulated (open cells) polyurethane cubes having edges of 2 cm and a nominal porosity of 45 pores per inch up to 50 % of its volume ;

b) an identical reactor was filled with 4 lamellae according to the invention up to 48 % of its volume, which lamellae were applied vertically in the reactor, wherein the backside of one lamella was positioned each time against the nap side of the subsequent lamella. Said lamellae have a backside of a thickness of 1 cm and the naps have a height of 2 cm. The lamellae consist of the same open cell polyurethane carrier material as in a). The concentration of the waste water introduced in both reactors was 7.1 g/l COD/l in average. The feeding was carried out discontinuously, viz. one time a day. A maximum volume charge (g COD/l.d) as well as a total COD reduction of at least 80 %, were aimed at.

Results :

1) using the cubus shaped polyurethane carriers 8.2 g COD/l-r.d (as a mean value) of the waste water was decomposed. Due to the fact that 50 % of the reactor volume was filled with cubes, this consequently implies a COD decomposition of 16.4 g/l polyurethane foam/day. The minimum attainable volume load for obtaining a COD reduction of 80 % was 9.4 g COD/l reactor/day.

2) using the lamellar polyurethane carriers 11.0 g COD (as an average value) was decomposed per liter reactor per day. With the filling of 48 % in the reactor this implies 22.9 g COD decomposition/l reactor/day. In the reactors containing lamellae a volume load of 13.2 g COD/l reactor/day was attainable and also a COD reduction of 80 %. There for the volume load attained with the lamellar reactor filling system had a superiority of 34 % as compared with the cubes at an equal COD reduction of 80 %.

Example II

A comparison was made between the starting-up rate of two reactors as described in example I.

Two identical reactors as in example I were filled now with the same amount of polyurethane carrier material. However, the first reactor contained lamellae having the same naps as in example I, which were placed with the backsides to the napsides. The other reactor was filled with cubes having edges of 2 cm.

5 % by volume of the reactors (again as described in the reference of example I) were filled with microbiological occulating material. The starting-up volume load was purposely kept very low and was identical for both reactors.

During the first week of the starting-up no clear difference was observed between the reactors of the two types at a mean value of 14 g COD/l reactor/d. During the second week of the starting-up an increased amount of 14 % COD was decomposed by the reactor filled with lamellae at a mean volume load of still 14 g COD/l.

Example III

A comparison was made with respect to the COD-decomposition and the biogas production/l reactor/day of a reactor according to example I packed with three layers lamellae having naps and one containing as much carrier material as in the reactor filled with lamellae, in which cubes of 2 cm were used. The packing of the cubes and the lamellae was carried out in such a way that this packing corresponded to that in full scale reactors.

The medium used was the same as in example I. At a volume load of 109 g COD/l reactor/day in both reactors, in the lamellar reactor 16 % more (as an average value) of COD/day was decomposed as compared with the reactor filled with cubes.

Consequently, the lamellar reactor evolves 48 l biogas/l reactor/day (corresponding to about 96 g COD decomposition/l reactor/day (as opposed to the cube reactor evolving 40.1 l biogas/l reactor/day (corresponding to about 80.2 g COD decomposition/l reactor/day). At the volume load used the lamellar reactor thus decomposed 88.1 % of the COD as opposed to the cube reactor, which decomposed 73.6 %.

Example IV

A lamellar reactor was compared with a cube reactor. In this example the medium used was a pre-acidified molasses solution of 30 g/COD/l, to which a part of minerals was added, as in the medium of AWWT. In this construction the reactor volumes of both reactors was 8 l and the feeding was carried out via a hose-pump having a controlable delivery, which pump was controlled by a timeclock. The excess of the medium was removed from the reactor by means of a gas tight siphon system. The waste water as such was recirculated by a second pump in the reactor.

The lamellar reactor contained 48 % carrier material of the lamellar nap type as described in example I whereas the second reactor contained 50 % cube carrier material.

The lamellar reactor decomposed in average 45 g COD/l/day of this waste water and reduced the COD to 25 % of the COD introduced (COD reduction of 75 %).

However, the corresponding cube reactor decomposes just 36 g COD/l/day, and provides a COD reduction of 75 % likewise. Thus, the difference between both reactors amounts to 20 % COD in favour of the lamellar reactor.

Claims

1. A process for carrying out microbiological fermentations wherein a nutrient liquid and, if required, gases are passed through a fixed system of open cell polyurethane foam units, characterized in using a system of at least 2 adjacent foam layers each having a flat main surface and an embossed main surface, the latter consisting of a pattern of protractions and recessed parts of substantially equal dimensions as set forth in Figure 1, wherein in the subsequent layers in each case the embossed surface of a foam layer is in contact with the flat surface of the adjacent foam layer.

2. The process according to claim 1, characterized by using at least 2 adjacent foam panels which are distributed over 10-100 %, preferably 40-80 % by volume, of the reactor capacity.

3. The process according to claim 1 or 2, characterized in that a rolled-up foam band (strip) is used.

4. The process according to claim 1-3, characterized in using a rolled-up foam band in which the embossed surface is directed outwardly.

5. The process according to claim 1-4, characterized by using a system of packed rolled-up foam bands in an upflow reactor, in which the flat surfaces are directed inwardly.

6. A device for carrying out microbiological fermentations wherein in a vessel a system of units of open cell polyurethane foam layers is fixed, characterized, in that the polyurethane foam layers have a flat main surface and an embossed main surface that consists of a regular pattern of protruding and recessed .parts of substantially equal dimensions and that in the subsequent layers in each case the embossed surface of a layer is in contact with the flat surface of the adjacent layer.

7. The device according to claim 6, characterized in that the system of units polyurethane foam layers consists of at least 2 adjacent foam panels.

8. The device according to claim 6 or 7, characterized in that the system of units of polyurethane foam layers consists of a rolled-up foam band wherein the embossed surface is directed outwardly.

9. The device according to claim 6-8, characterized in that an upflow reactor contains a system of packed rolled-up foam bands wherein the flat surfaces are directed inwardly.

**Patentansprüche**

1. Verfahren zur Durchführung von mikrobiologischen Fermentationen, bei welchen eine Nährmittelflüssigkeit und, falls erforderlich, Gase durch ein fixiertes System aus offenzelligen Polyurethanschaumeinheiten geleitet werden, dadurch gekennzeichnet, daß ein System aus wenigstens zwei benachbarten Schaumschichten verwendet wird, von denen jede eine flache Hauptoberfläche und eine hohlgeprägte Oberfläche aufweist, wobei die letztere aus einem Muster aus Hervorhebungen und ausgesparten Teilen mit im wesentlichen gleichen Abmessungen, wie aus Figur 1 hervorgeht, aufweist, und wobei in den aufeinanderfolgenden Schichten in jedem Falle die hohlgeprägte Schaumschicht in Kontakt mit der flachen Oberfläche der benachbarten Schaumschicht steht.

2. Verfahren nach Anspruch 1, dadurch gekennzeichnet, daß wenigstens zwei benachbarte Schaumplatten verwendet werden, die über 10 bis 100 % und vorzugsweise 40 bis 80 Vol.-% der Reaktorkapazität verteilt sind.

3. Verfahren nach Anspruch 1 oder 2, dadurch gekennzeichnet, daß ein aufgerolltes Schaumband (-streifen) verwendet wird.

4. Verfahren nach Anspruch 1 bis 3, dadurch gekennzeichnet, daß ein aufgerolltes Schaumband verwendet wird, in welchem die hohlgeprägte Oberfläche nach außen gerichtet ist.

5. Verfahren nach Anspruch 1 bis 4, dadurch gekennzeichnet, daß ein System aus gepackten aufgerollten Schaumbändern in einem Reaktor mit nach oben gerichteter Strömung verwendet wird, in welchen die flachen Oberflächen nach innen gerichtet sind.

6. Vorrichtung zur Durchführung von mikrobiologischen Fermentationen, wobei in einem Gefäß ein System aus Einheiten aus offenzelligen Polyurethanschaumschichten fixiert ist, dadurch gekennzeichnet, daß die Polyurethanschaumschichten eine flache Hauptoberfläche und eine hohlgeprägte Oberfläche, die aus einem regelmäßigen Muster aus hervorstehenden und ausgesparten Teilen von im wesentlichen gleichen Dimensionen besteht, aufweist, und daß in den folgenden Schichten in jedem Falle die hohlgeprägte Oberfläche einer Schicht in Kontakt mit der flachen Oberfläche der benachbarten Schicht steht.

7. Vorrichtung nach Anspruch 6, dadurch gekennzeichnet, daß das System von Einheiten von Polyurethanschaumschichten aus wenigstens zwei benachbarten Schaumplatten besteht.

8. Vorrichtung nach Anspruch 6 oder 7, dadurch gekennzeichnet, daß das System aus Einheiten von Polyurethanschaumschichten aus einem aufgerollten Schaumband besteht, wobei die hohlgeprägte Oberfläche nach außen gerichtet ist.

9. Vorrichtung nach Anspruch 6 bis 8, dadurch gekennzeichnet, daß ein Reaktor mit nach oben gerichteter Strömung ein System von gepackten aufgerollten Schaumbändern enthält, wobei die flachen Oberflächen nach innen gerichtet sind.

**Revendications**

1. Procédé pour réaliser des fermentations microbiologiques, dans lequel on fait passer un liquide nutritif et, si nécessaire, des gaz à travers un ensemble fixe d'unités en mousse de polyuréthane à cellules ouvertes, caractérisé en ce que l'on utilise un ensemble d'au moins deux couches de mousse adjacentes présentant chacune une surface principale plane et une surface principale bosselée, cette dernière consistant en un motif de protubérances et de parties évidées ayant des dimensions sensiblement égales comme représenté à la figure 1, dans lequel la surface bosselée d'une couche de mousse parmi les couches consécutives est dans chaque cas en contact avec la surface plane de la couche de mousse adjacente.

2. Procédé conforme à la revendication 1, caractérisé en ce que l'on utilise au moins deux panneaux de mousse adjacents qui sont répartis sur un volume compris entre 10 et 100 %, de préférence entre 40 et 80 % de la capacité du réacteur.

3. Procédé conforme à l'une des revendications 1 ou 2, caractérisé en ce que l'on utilise une bande (ruban) de mousse enroulée.

4. Procédé conforme à l'une des revendications 1 à 3, caractérisé en ce que l'on utilise une bande de mousse enroulée dont la surface bosselée est dirigée vers l'extérieur.

5. Procédé conforme à l'une des revendications 1 à 4, caractérisé en ce que l'on utilise un ensemble de bandes de mousse enroulées et chargées dans un réacteur à courant ascendant, les surfaces planes étant dirigées vers l'intérieur.

6. Dispositif pour réaliser des fermentations microbiologiques dans lequel un ensemble d'unités de couches de mousse de polyuréthane à cellules ouvertes est fixe dans un récipient, caractérisé en ce que les couches de mousse de polyuréthane présentent une surface principale plane et une surface principale bosselée qui consiste en un motif régulier de parties protubérantes et de parties évidées de dimensions sensiblement égales, et en ce que la surface bosselée d'une couche parmi les couches successives est dans chaque cas en contact avec la surface plane de la couche adjacente.

7. Dispositif conforme à la revendication 6, caractérisé en ce que l'ensemble des unités de couches de mousse de polyuréthane consiste en au moins deux panneaux de mousse adjacents.

8. Dispositif conforme à l'une des revendications 6 ou 7, caractérisé en ce que l'ensemble des unités de couches de mousse de polyuréthane consiste en une bande de mousse enroulée dont la surface bosselée est dirigée vers l'extérieur.

9. Dispositif conforme à l'une des revendications 6 à 8, caractérisé en ce qu'un réacteur à écoulement ascendant contient un ensemble de bandes de mousse enroulées et chargées dont les surfaces planes sont dirigées vers l'intérieur.

# fig-1

# fig-2

fig-3